# EUROPÄISCHE PATENTSCHRIFT

(11) **EP 0 711 774 B1**
(45) Veröffentlichungstag und Bekanntmachung des Hinweises auf die Patenterteilung: **13.02.2002**
(21) Anmeldenummer: 95117409.3
(22) Anmeldetag: 06.11.1995
(51) Int. Cl.: C07D 501/00, A61K 31/545

(54) **Neue kristalline Cephem-Säureadditionssalze und Verfahren zu ihrer Herstellung**
Crystalline cephem-addition salts and process for their preparation
Sels d'addition cristallin de céphem et procédé pour leur préparation

(30) Priorität: 10.11.1994 DE 4440141
(43) Veröffentlichungstag der Anmeldung: 15.05.1996
(73) Patentinhaber: Aventis Pharma Deutschland GmbH, 65929 Frankfurt am Main (DE)
(72) Erfinder: Lattrell, Rudolf, Dr., D-61462 Königstein (DE); Dürckheimer, Walter, Dr., D-65795 Hattersheim (DE); Schmid, Peter, Dr., D-65620 Waldbrunn-Lahr (DE)

(56) Entgegenhaltungen:
- EP-A- 0 112 550
- EP-A- 0 125 576
- DE-A- 3 706 020
- THE JOURNAL OF ANTIBIOTICS, Bd.XLI, Nr.10, Oktober 1988 Seiten 1374 - 1394 RUDOLF LATTRELL ET AL

## Beschreibung

Die Erfindung betrifft neue kristalline Cephem-Säureadditionssalze, die sich durch eine besondere Schwerlöslichkeit auszeichnen, Verfahren zu ihrer Herstellung sowie ihre Verwendung als Arzneimittel.

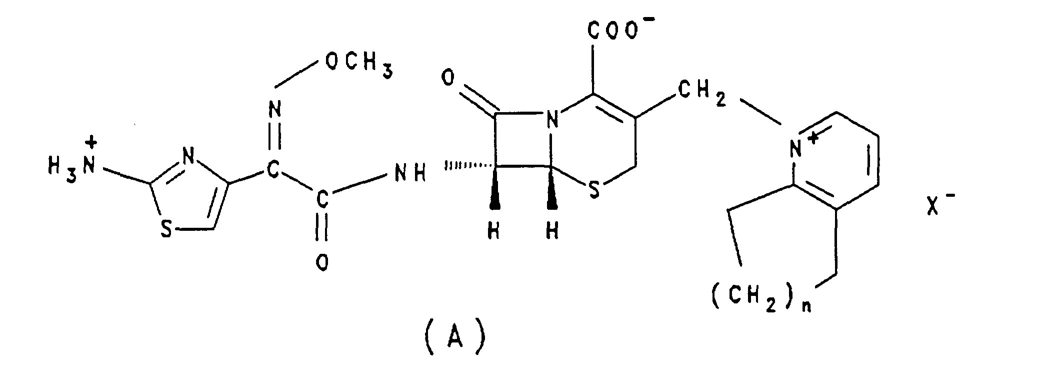

In den deutschen Patentanmeldungen DE 3 248 281 (US 4 609 653) (für n = 1) sowie DE 3 706 020 (US 4 845 087) (für n = 2) werden kristallisierte Säureadditionssalze der Formel A vorgeschlagen, die zur Behandlung von bakteriellen Infektionen verwendet werden. Ihre Wirkstoffspiegel im Plasma sind jedoch insbesondere für den Einsatz auf dem Veterinärsektor nicht befriedigend lang.

Eine Aufgabe dieser Erfindung ist es daher, Salze mit einer im Vergleich zu den bekannten Salzen deutlich verlängerten terminalen Halbwertszeit und damit längeren Wirkstoffspiegeln im Plasma und Gewebe zu finden. Diese Aufgabe wird überraschenderweise gelöst durch Salze der Formel I, 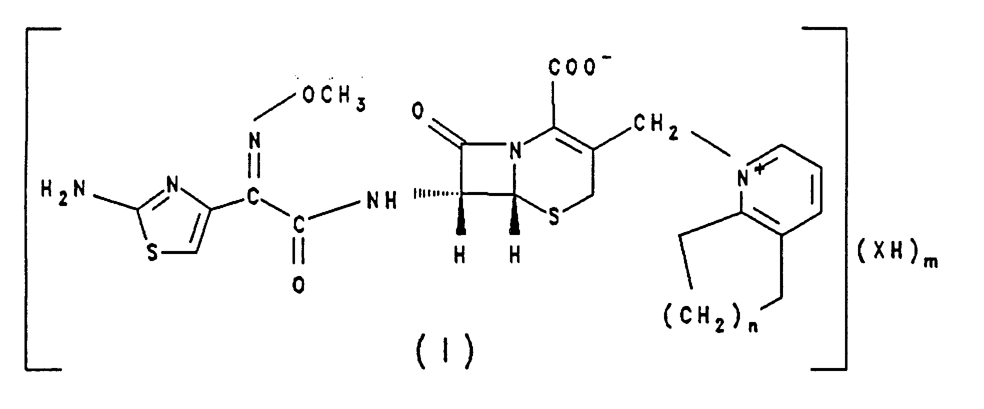 worin
n gleich 1 oder 2 und
m 0,4 - 2,6 ist und wobei
X das Anion einer phenolischen Carbonsäure der Formeln II a-d 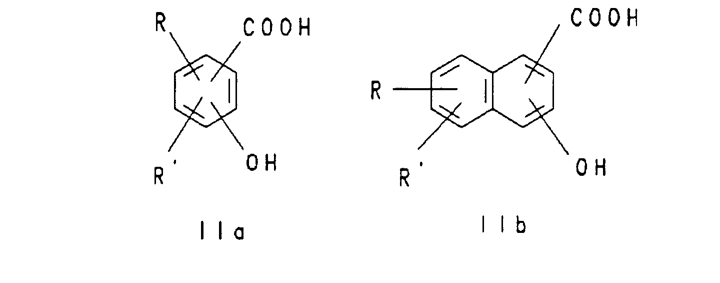 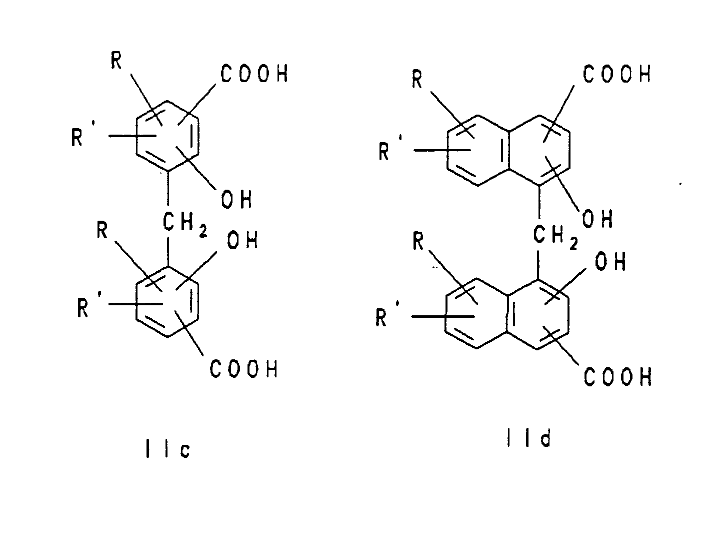
oder das Anion einer Hydroxyphenylessigsäure der Formel III, 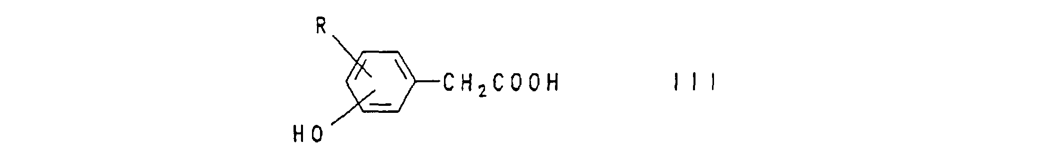 oder das Anion einer Hydroxyzimtsäure der Formel IV, 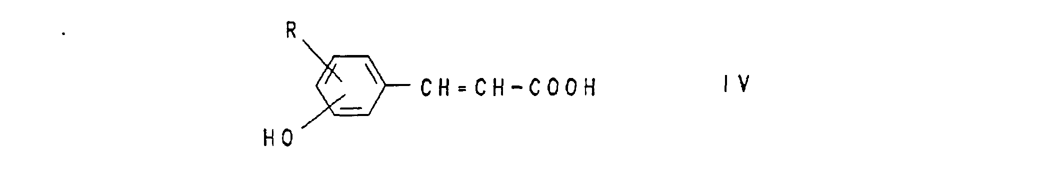 oder das Anion einer Hydroxyhippursäure der Formel V, 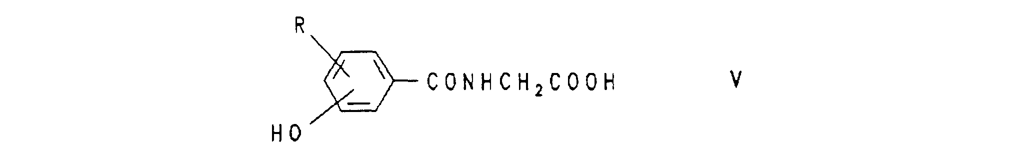 darstellt, wobei R und R' unabhängig voneinander für Wasserstoff, Carboxy, Hydroxy, Halogen, einen geradkettigen oder verzweigten gesättigten oder ungesättigten aliphatischen C₁-C₅-Alkylrest oder C₁-C₅- Alkyloxyrest stehen und wobei X auch X/2 im Falle einer zweiprotonigen Säure bedeuten kann.

Bevorzugte Verbindungen der Formel I enthalten die Carbonsäuren der Formeln II- V, in denen R und R' Wasserstoff, Carboxy, Hydroxy, Methyl oder Methoxy bedeutet und in denen m für 0,5 - 2 steht.

Besonders bevorzugt sind beispielsweise die folgenden Säuren:
Dihydroxybenzoesäuren, wie 2.4-, 2.5- oder 3,5-Dihydroxybenzoesäure Trihydroxybenzoesäuren, wie Gallussäure,
Hydroxydicarbonsäuren, wie 4-Hydroxyisophthalsäure,
Hydroxynaphthalincarbonsäuren, wie 2-Hydroxynaphthalin-1-carbonsäure oder 6-Hydroxynaphthalin-1-carbonsäure,
Methylen-bis-hydroxybenzoesäuren, wie 5.5'-Methylen-bis-4-hydroxybenzoesäure,
Methylen-bis-hydroxynaphthoesäuren, wie Embonsäure, Hydroxyzimtsäuren wie Ferulasäure,
Hydroxyhippursäuren wie Salicylursäure.

Anhand der nachfolgenden Formel I' 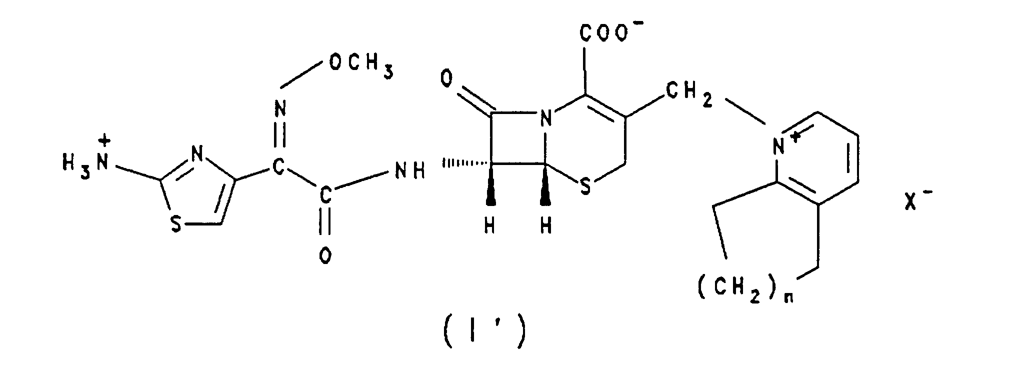 soll eine erfindungsgemäße Verbindung mit m = 1 vereinfacht beispielhaft dargestellt werden. Die Darstellung ist vereinfachender Natur weil die tatsächliche Struktur in dem Salz und vor allem eine exakte Lokalisierung der Ladung nicht ermittelt sind. Die Verbindungen mit m ca. 1 sind besonders bevorzugt.

Der Begriff XH in der obengenannten Formel I kann selbstverständlich auch das Anion einer zweiprotonigen Säure umfassen, woraus folgt, daß der Begriff dann strenggenommen X_{1/2}H bedeuten müßte.

Die Erfindung umfaßt weiterhin Verfahren zur Herstellung der Salze der Formel I, die dadurch gekennzeichnet sind, daß man
1. ein in Wasser lösliches Salz der Formel I, beispielsweise ein Dihydrochlorid, Dihydrojodid oder ein Sulfat, mit Salzen der Carbonsäuren der Formeln II, III, IV oder V umsetzt, oder
2. ein Cephem-Betain der Formel VI 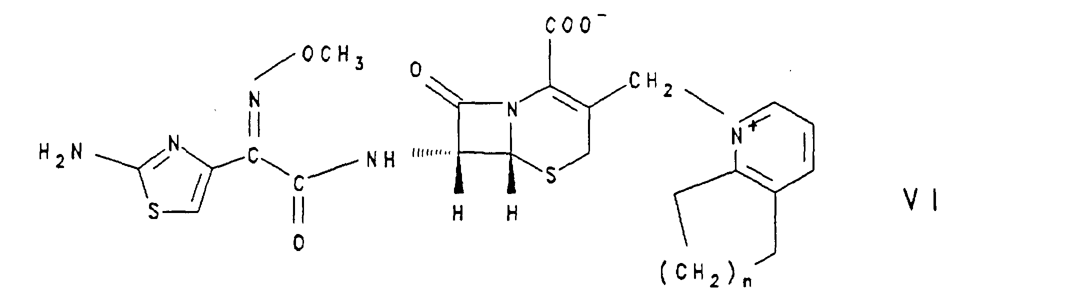 worin n die zu Formel I genannte Bedeutung hat, mit den Carbonsäuren der Formeln II, III, IV oder V umsetzt.

Die Herstellung der in Wasser löslichen Salze der Formel I wird in den oben zitierten Patentanmeldungen, das Betain der Formel VI in dem Europäischen Patent EP 64740 (US 5 071 979) beschrieben.

Nach dem Verfahren 1 wird zur Lösung eines dieser Salze I in Wasser ein in Wasser lösliches Salz der Carbonsäuren II, III, IV oder V, beispielsweise ein Natrium-, Kalium- oder Magnesiumsalz zugegeben, wobei das zugrundeliegende Anion ein in Wasser schwer lösliches Säureadditionssalz der Formel I bildet. Diese Salze können in Substanz oder in wäßriger Lösung oder in Gemischen aus Wasser und mit Wasser mischbaren organischen Lösungsmitteln, wie z.B. Methanol, Ethanol, Isopropanol, Aceton, Tetrahydrofuran, DMSO, zugegeben werden.

Die Bildung der Salze der Formel I wird bei Temperaturen zwischen - 10° und + 60°C, vorzugsweise zwischen + 5° und + 30°C vorgenommen. Die Kristallisation der Salze erfolgt spontan während der Zugabe der Salze der Carbonsäuren II - V, die in äquimolarer Menge bis zu einem ca. 2,6-fachen Überschuß verwendet werden.

Das Verfahren kann auch so durchgeführt werden, daß man die Salze der Carbonsäuren II - V vorlegt und das in Wasser gelöste Säureadditionssalz I zugibt.

Die erfindungsgemäßen Salze der Formel I werden durch Filtration oder Zentrifugation isoliert und in üblicher Weise, beispielsweise durch Gefriertrocknung oder mit Hilfe eines wasserentziehenden Mittels, wie z.B. Kaliumhydroxid oder Phosphorpentoxid, getrocknet. Beimengungen, z.B. von überschüssigen Carbonsäuren II - V können gegebenenfalls durch Ausrühren mit einem mit Wasser mischbaren organischen Lösungsmittel, wie Aceton, Ethanol und Isopropanol, entfernt werden.

Es lassen sich natürlich durch entsprechende Wahl der Ausgangskomponenten schwerlösliche Salze erhalten, die einen stöchiometrischen Überschuß oder Unterschuß der Carbonsäure enthalten, z.B. 0,4 Mol bis 2,6 Mol Säure pro Mol Betain der Formel VI. Beispielsweise läßt sich in dem Salz von Beispiel 1 durch Behandeln mit organischen Lösungsmitteln, wie z.B.Aceton oder Ethanol der Säureanteil vermindern.

Nach dem Verfahren 2 wird eine Lösung der Verbindung VI in Wasser mit Carbonsäuren der Formeln II - V versetzt. Diese Säuren können in Substanz oder in Lösung, z.B. in mit Wasser mischbaren organischen Lösungsmitteln wie Aceton, Ethanol oder Isopropanol, zugegeben werden. Die Reaktionsführung und Kristallisation erfolgt wie bei Verfahren 1 beschrieben.

Die erfindungsgemäß erhaltenen Verbindungen der allgemeinen Formel I zeigen sehr gute antibakterielle Wirksamkeit sowohl gegen grampositive als auch gramnegative einschließlich gegen penicillinase- und cephalosporinasebildende Keime. Da sie zudem günstige toxikologische und pharmakologische Eigenschaften zeigen, stellen sie wertvolle Chemotherapeutika dar.

Die Erfindung betrifft somit auch Arzneimittel zur Behandlung von mikrobiellen Infektionen bei Säugern, sowohl bei Menschen als auch bei Tieren, sowie bei Vögeln und in der Aquakultur, die durch einen Gehalt an den erfindungsgemäßen physiologischen verträglichen Säureadditionssalzen charakterisiert sind. Sie können auch in Kombination mit anderen Wirkstoffen, beispielsweise aus der Reihe der Penicilline, Cephalosporine oder Aminoglykoside zur Anwendung kommen.

Die Verbindungen der allgemeinen Formel I können subcutan, intramuskulär, bei Tieren auch noch intratracheal oder lokal, wie z.B. in das Euter von milchgebenden Tieren verabfolgt werden.

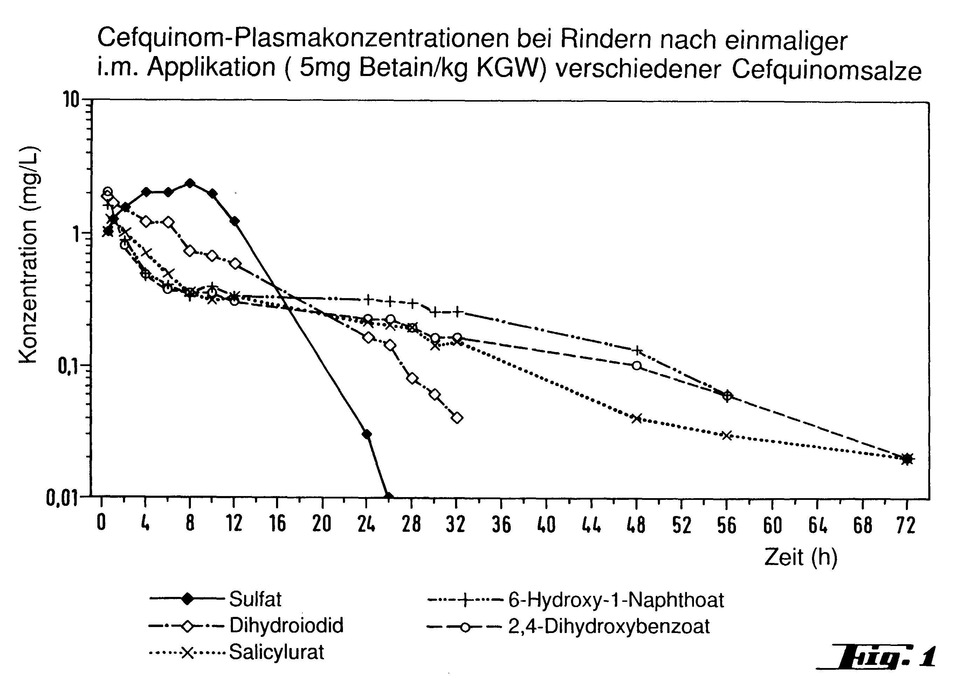

Dabei zeigen die erfindungsgemäßen Säureadditionssalze im Vergleich zu den bereits bekannten in Wasser leichter löslichen Salze bemerkenswerte Vorteile, die auf ihrer Schwerlöslichkeit und Pharmakokinetik im Tier beruhen. Sie werden z.B. bei Rindern nach intramuskulärer (i.m.) Applikation verzögert resorbiert, die terminalen Halbwertszeiten (Tabelle 1) sowie die Wirkstoffspiegel in Plasma sind gegenüber den bekannten Salzen deutlich verlängert. In Tabelle 1 sind für erfindungsgemäße Verbindungen die terminalen Halbwertszeiten (t_{½}) nach Einmalinjektion (i.m.) von 5 mg Betain VI (n = 2) pro kg Körpergewicht bei Rindern zusammengefaßt. In Figur 1 sind die Plasmakonzentration-Zeit-Kurven von Rindern nach Einmalapplikation verschiedener Cefquinomsalze I (n = 2) dargestellt (i.m., 5 mg Betain/kg KGW). Als Standard dienen die bekannten Salze Sulfat und Dihydrojodid aus DE 3 706 020. Von den erfindungsgemäßen Säureadditionssalzen wurden exemplarisch das 6-Hydroxy-1-naphthoat (Beispiel 1), das Cefquinom-2.4-Dihydroxybenzoat (Beispiel 11), sowie das Salicylurat (Beispiel 23) dargestellt.

Auch die oben beschriebenen Salze, die vom Verhältnis 1:1 abweichen, sind als Depotformen einsetzbar und erlauben die Pharmakokinetik beim Tier einzustellen.

**Tabelle 1**

| Terminale Halbwertszeiten (t_{½}) von erfindungsgemäßen Cefquinomsalzen bei Rindern nach einmaliger i.m.-Applikation von 5 mg Betain/kg KGW | | | | | |
|---|---|---|---|---|---|
| Beispiel Nr. | t_{½} h | Beispiel Nr. | t_{½} h | Beispiel Nr. | t_{½} h |
| Sulfat | 2.15 | 11 | 16.20 | 18 | 13.00 |
| Dihydrojodid | 4.19 | 12 | 15.20 | 19 | 7.07 |
| 1 | 9.20 | 13 | 9.21 | 20 | 9.39 |
| 2 | 18.8 | 15 | 11.1 | 21 | 9.96 |
| 6 | 5.87 | 16 | 9.16 | 22 | 8.77 |
| 9 | 15.6 | 17 | 17.8 | 23 | 12.70 |

Arzneimittel, die eine oder mehrere Verbindungen der allgemeinen Formel I als Wirkstoff enthalten und ebenfalls zum Gegenstand der vorliegenden Erfindung gehören, können hergestellt werden, indem man die Verbindungen der Formel I mit einem oder mehreren physiologisch verträglichen Trägerstoffen, Verdünnungsmitteln oder Puffersubstanzen vermischt, und in eine zur parenteralen oder lokalen Applikation geeignete Zubereitungsform bringt.

Als Verdünnungsmittel seien beispielsweise erwähnt Polyglykole, Dimethylsulfoxid, N-Methylpyrrolidon, N,N-Dimethylacetamid, Ethanol und Wasser. Puffersubstanzen sind beispielsweise organische Verbindungen, wie z.B. N',N'-Dibenzylethylendiamin, Diethanolamin, Ethylamin, Tris(hydroxymethyl)aminomethan, oder anorganische Verbindungen, wie z.B. Phosphatpuffer, Natriumbicarbonat, Natriumcarbonat. Für die parenterale Applikation kommen vorzugsweise Suspensionen oder Lösungen in Wasser mit oder ohne Puffersubstanzen in Betracht.

Geeignete Dosen der Verbindungen der allgemeinen Formel I liegen bei der Verabreichung an Menschen bei etwa 0.4 bis 20 g/Tag, vorzugsweise bei 0.5 bis 4 g/Tag für einen Erwachsenen von etwa 60 kg Körpergewicht.

Es können Einzel- oder im allgemeinen Mehrfachdosen verabreicht werden, wobei die Einzeldosis den Wirkstoff in einer Menge von etwa 50 bis 1000 mg, vorzugsweise von etwa 1200 bis 500 mg enthalten kann.

Bei einer Verabreichung an Tieren kommen prinzipiell alle Säugetiere sowie Vögel und Fische in Betracht, im Hinblick auf ihre Bedeutung insbesondere Haus- und Nutztiere. Die Dosierung variiert bei den einzelnen Tierarten und kann beispielsweise zwischen etwa 1,8 und 150, vorzugsweise zwischen etwa 5 und 50 mg/kg Körpergewicht des Tieres liegen.

Die folgenden Ausführungsbeispiele für erfindungsgemäß herstellbare Säureadditionsverbindungen des Cephem-Betains der Formel VI (n = 1: Cefpirom, n = 2: Cefquinom) sowie der Inhalt der Patentansprüche dienen zur weiteren Erläuterung der Erfindung, schränken sie jedoch nicht darauf ein.

### Beispiel 1

### Cefquinom-6-Hydroxy-1-naphthoat

### Verfahren 1:

188.0 g (0.3 mol) Cefquinom-Sulfat werden in 7.4 I Wasser bei Raumtemperatur gelöst. Unter Eiskühlung wird innerhalb 30 Minuten eine Lösung von 101.6 g (0.54 mol) 6-Hydroxy-1-naphthoesäure in 300 ml 2N Natronlauge und 500 ml Wasser zugetropft. Während des Zutropfens bildet sich eine Suspension, die Temperatur fällt auf 15°C. Es wird 45 Minuten im Eisbad nachgerührt, der Niederschlag abgesaugt, in 700 ml Eiswasser suspendiert, wieder abgesaugt und mit 300 ml Eiswasser gewaschen. Der feuchte Niederschlag wird 3 Tage gefriergetrocknet.
Ausbeute: 225 g Titelverbindung + 6-Hydroxy-1-naphthoesäure

Zur Entfernung der überschüssigen Säure wird dreimal in jeweils 1.2 l Aceton suspendiert, 10 Minuten gerührt, abgesaugt und mit je 200 ml Aceton gewaschen. Nach Trocknen an der Luft erhält man 173.5 g (0.24 mol) der Titelverbindung als farbloses feinkristallines Produkt.
Zers.: 190 - 210°C.

¹H-NMR (270 MHz, DMSO-d₆) δ = 1.65-2.0 (4H, m); 2.85-3.18 (4H, m); 8 Cyclohexen-H; 3.10 und 3.40 (2H, AB, J=18 Hz, SCH₂), 3.80 (3H, s, OCH₃); 5.03 (1H, d, J=5 Hz, 6-H); 5.33 und 5.42 (2H, AB, J=15 Hz 3'-CH₂); 5.63 (1H, dd, J=5.8 Hz, 7-H); 6.72 (1H, s, Thiazol-H); 7.21 (4H, m, NH₂ und 2 arom. H); 7.43 (1H, dd, J=7 Hz, 1 arom. H); 7.90 (3H, m, 1 Py-H und 2 arom. H); 8.26 (1H, d, J=7 Hz, Py-H); 8.70 (1H, d, J=8 Hz, 1 arom. H); 9.21 (1H, d, J=7 Hz, Py-H); 9.55 (1H, d, J=8 Hz, CONH)

In Analogie zu Beispiel 1 werden die Cefquinomsalze I, n = 2, der Tabelle 2 (Beispiele 2-23, Seite 11-15) aus Cefquinom-Sulfat und der Carbonsäure X hergestellt.

In Analogie zu Beispiel 1 werden die Cefpiromsalze I, n = 1, der Tabelle 3 (Beispiele 24-28, Seite 16) aus Cefpirom-Sulfat und der Carbonsäure X hergestellt.

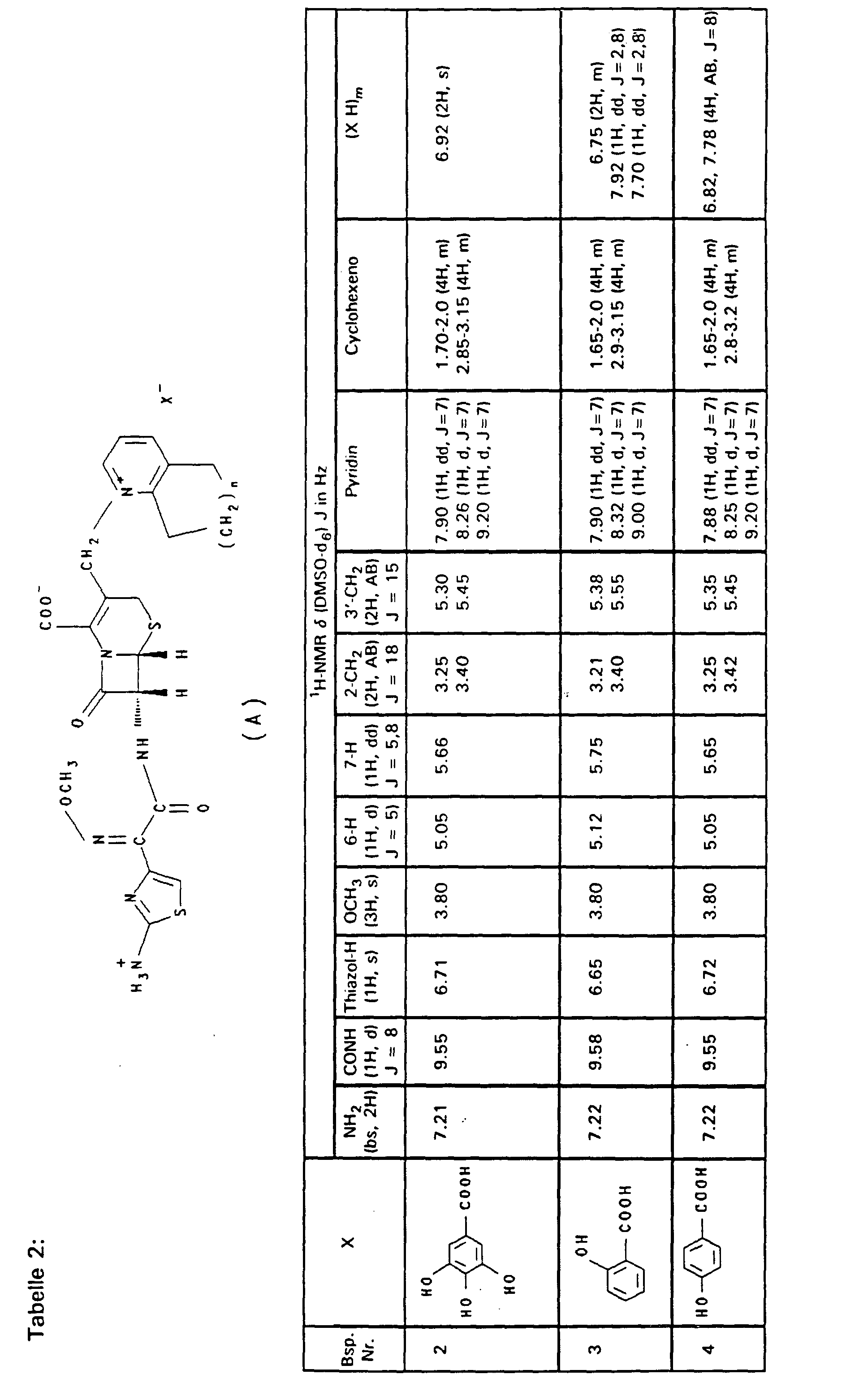

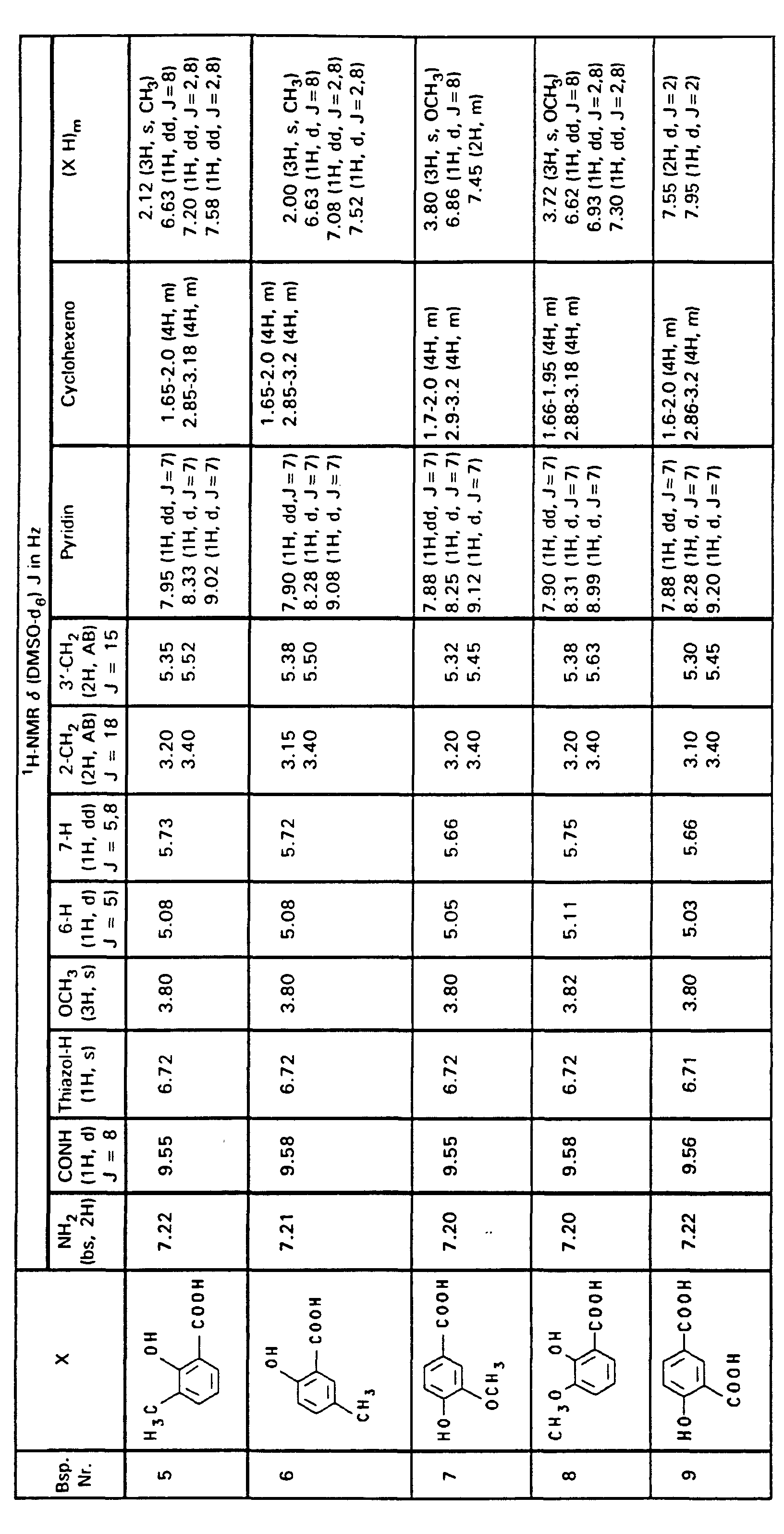

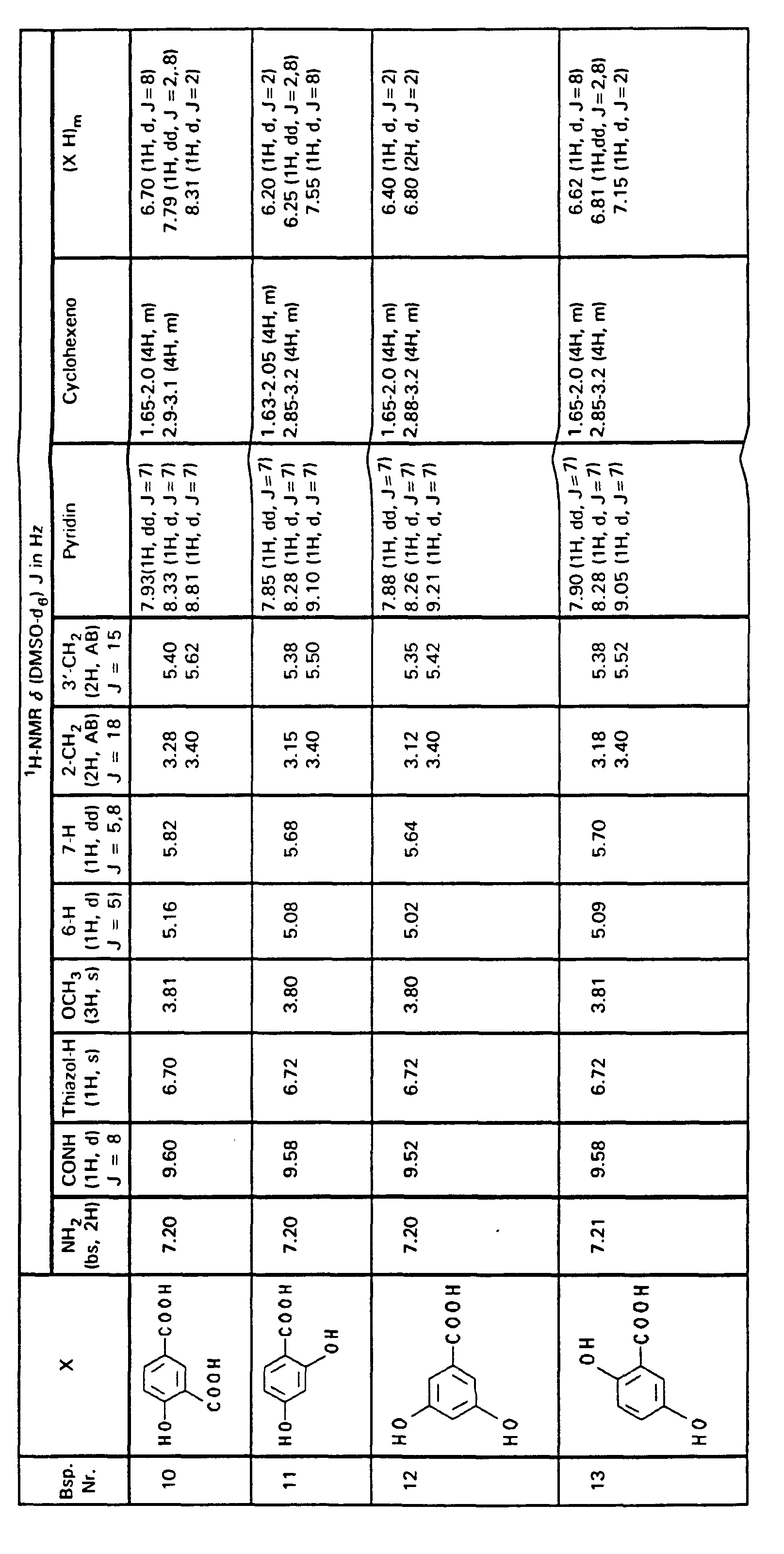

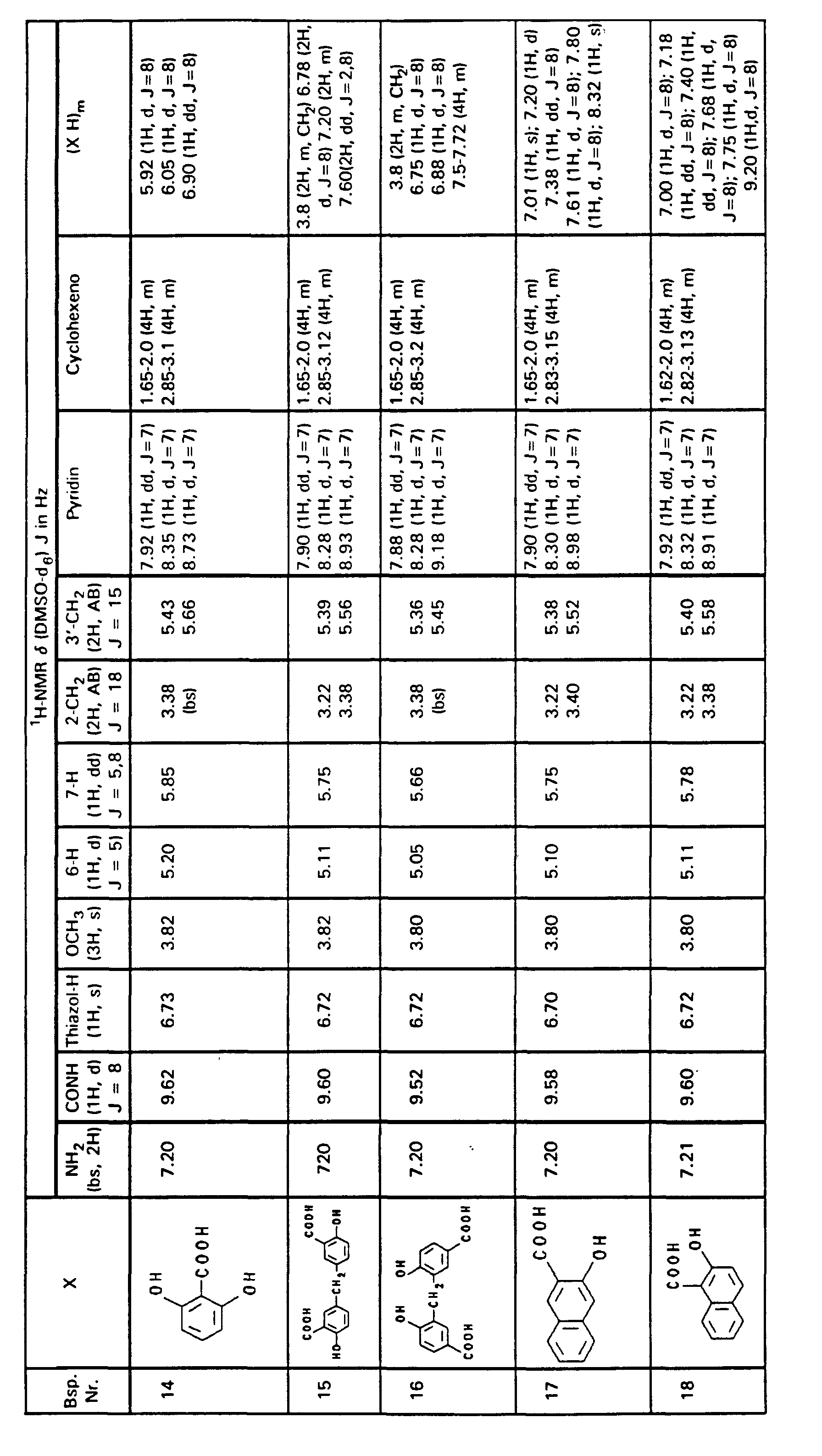

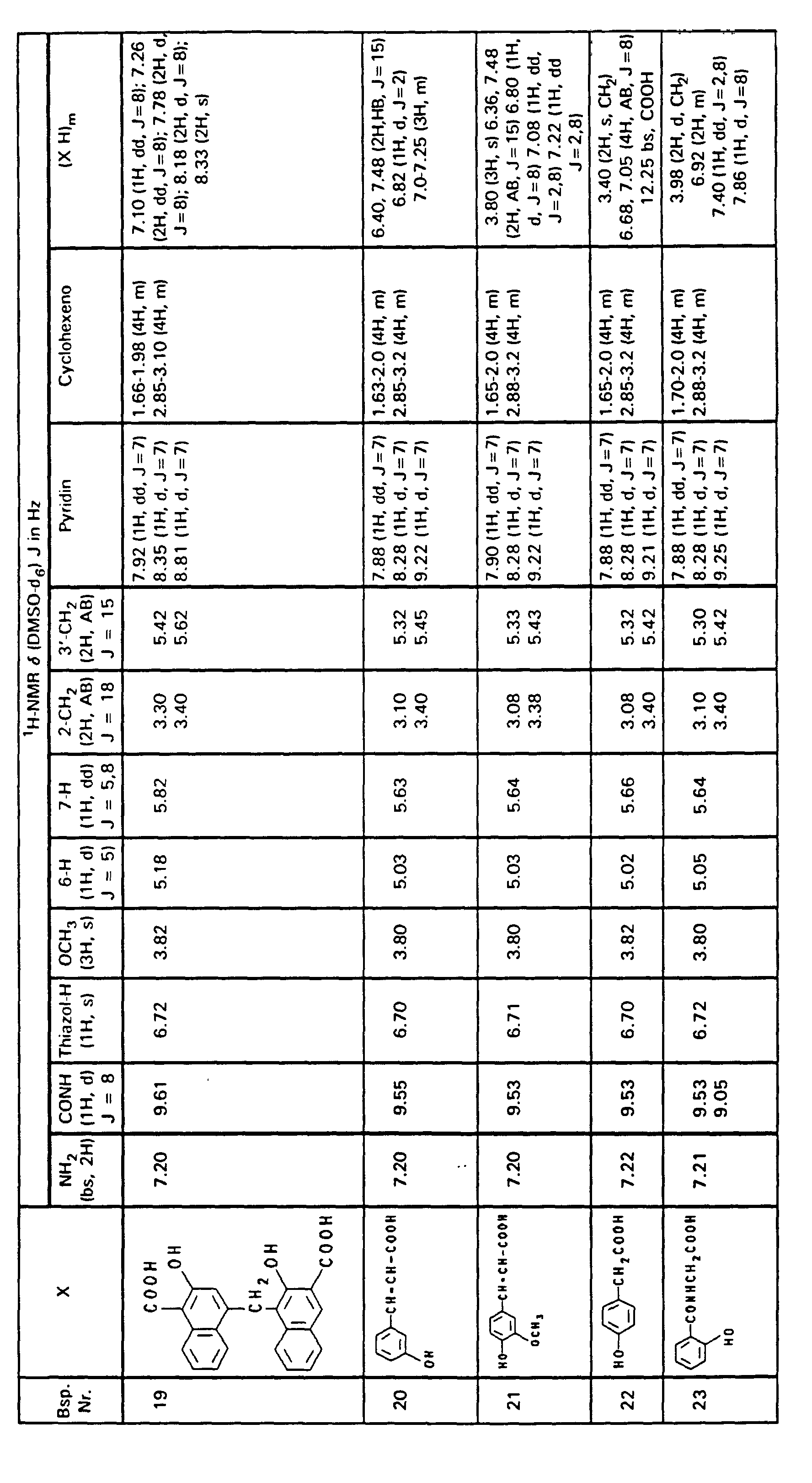

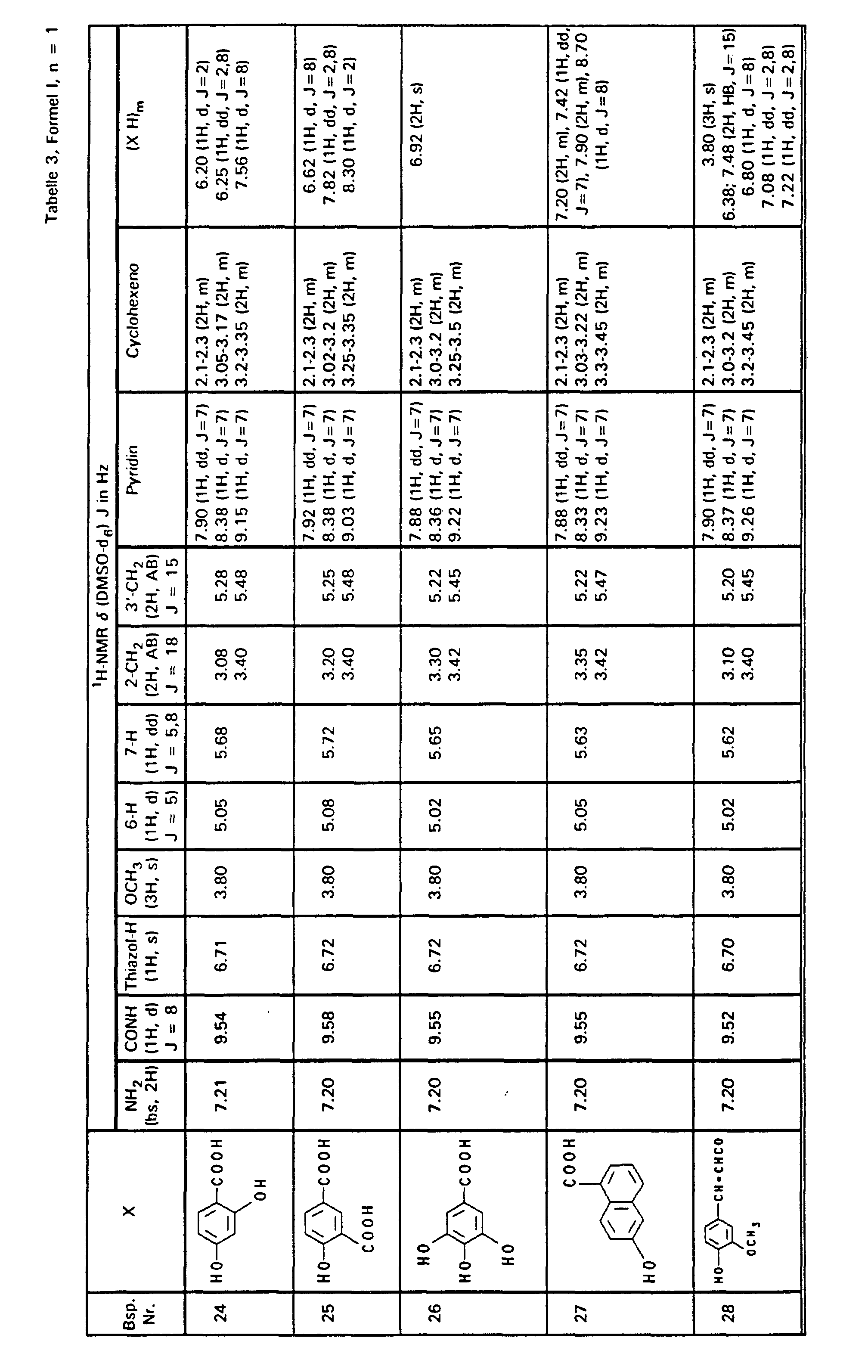

### Beispiel 29

### Cefquinom-2,4-Dihydroxybenzoat

### Verfahren 2:

Zur Lösung von 3.14 g (5 mmol) Cefquinom-Sulfat in 125 ml Wasser werden bei Raumtemperatur 5 ml 2N Natronlauge zugegeben. Die gebildete Betain-Lösung wird auf 15°C gekühlt. Innerhalb 5 Minuten wird sodann eine Lösung von 1.39 g (9 mmol) 2.4-Dihydroxybenzoesäure in 10 ml Aceton zugetropft. Die erhaltene Suspension wird 5 Stunden im Eisbad gerührt, der Niederschlag abgesaugt, mit 5 ml Eiswasser gewaschen und über Phosphorpentoxid im Vakuum getrocknet. Das Rohprodukt (1.98 g) wird mit 40 ml Aceton 2 Stunden verrührt, der Niederschlag abgesaugt, mit 10 ml Aceton gewaschen und über P₂O₅ im Vakuum getrocknet.
- Ausbeute:: 1.873 g (2.74 mmol) farbloses kristallines Produkt
- Zers.:: 175 - 185°C

Die Verbindung ist in allen Eigenschaften mit der Verbindung aus Beispiel 11 identisch.

### Beispiel 30

### Cefquinom-2,3-Dihydroxy-5-methylbenzoat

In Analogie zu Beispiel 29 werden aus 3.14 g (5 mmol) Cefquinom-Sulfat und 1.51 g (9 mmol) 2,3-Dihydroxy-5-methylbenzoesäure 1.81 g (2.6 mmol) der Titelverbindung als hellbraunes kristallines Produkt erhalten.

¹H-NMR (270 MHz, DMSO-d₆): δ = 1.63-1.98 (4H, m); 2.12 (3H,s,CH₃); 2.86-3.18 (4H, m); 3.10 und 3.40 (2H, AB, J=18 Hz, SCH₂); 3.81 (3H, s, OCH₃); 5.10 (1H, d, J=5 Hz, 6-H); 5.34 und 5.55 (2H, AB, J=15 Hz, 3'-CH₂); 5.75 (1H, dd, J=5.8 Hz, 7-H); 6.69 (1H, d, J=2 Hz, arom. H); 6.72 (1H, s, Thiazol-H); 7.00 (1H, d, J=2 Hz, arom. H); 7.20 (2H, bs, NH₂); 7.90 (1H, dd, J=7 Hz, Py-H); 8.30 (1H, d, J=7 Hz, Py-H); 9.00 (1H, d, J=7 Hz, Py-H); 9.58 (1H, d, J=8 Hz, CONH)

## Patentansprüche

1. Verbindungen der Formel I 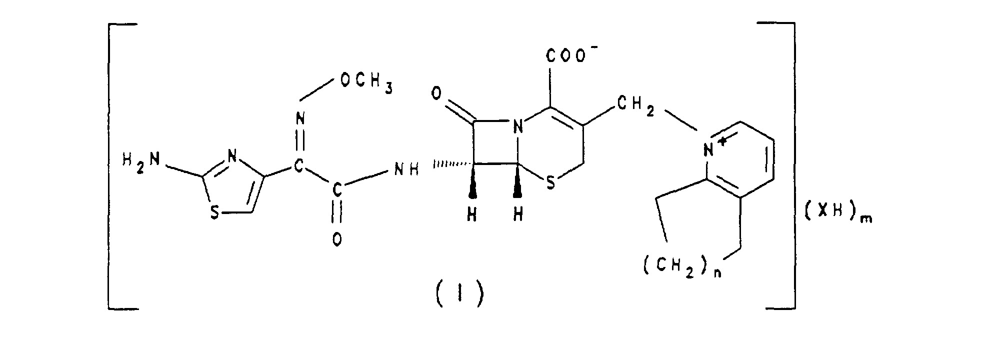 worin
n gleich 1 oder 2 und
m 0,4 - 2,6 ist und wobei
X das Anion einer phenolischen Carbonsäure der Formeln II a-d 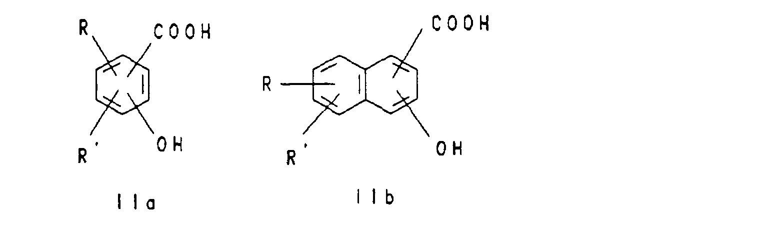 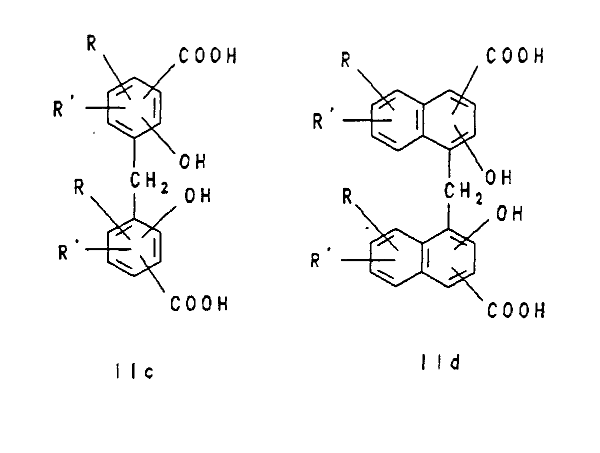
oder das Anion einer Hydroxyphenylessigsäure der Formel III, 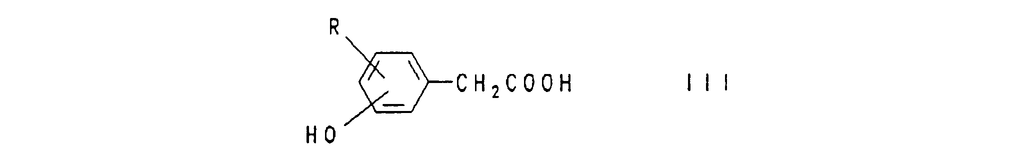 oder das Anion einer Hydroxyzimtsäure der Formel IV, 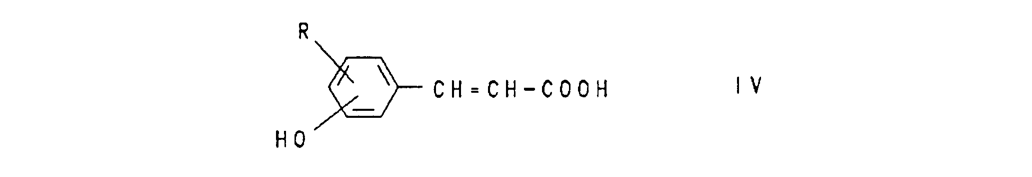 oder das Anion einer Hydroxyhippursäure der Formel V, 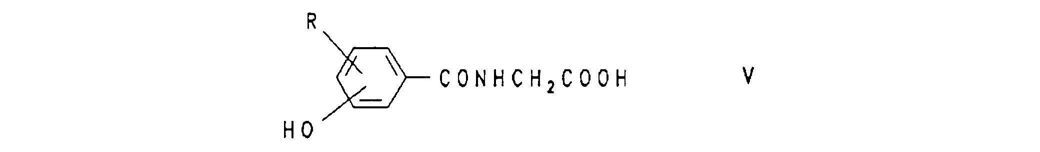 darstellt, wobei R und R' unabhängig voneinander für Wasserstoff, Carboxy, Hydroxy, Halogen, einen geradkettigen oder verzweigten gesättigten oder ungesättigten aliphatischen C₁-C₅-Alkylrest oder C₁-C₅- Alkyloxyrest stehen und wobei X auch X/2 im Falle einer zweiprotonigen Säure bedeuten kann.

2. Verbindungen der Formel I gemäß Anspruch 1, **dadurch gekennzeichnet, daß** X das Anion einer Carbonsäure der Formeln II- V darstellt, in denen R und R'unabhängig voneinander Wasserstoff, Carboxy, Hydroxy, Methyl oder Methoxy bedeuten und wobei m für 0,5 - 2 steht.

3. Verbindungen der Formel I gemäß den Ansprüchen 1 oder 2, **dadurch gekennzeichnet, daß** X das Anion einer Verbindung ausgewählt aus der folgenden Gruppe von Säuren ist:
Dihydroxybenzoesäuren, wie 2.4-, 2.5- oder 3.5-Dihydroxybenzoesäure Trihydroxybenzoesäuren, wie Gallussäure,
Hydroxydicarbonsäuren, wie 4-Hydroxyisophthalsäure,
Hydroxynaphthalincarbonsäuren, wie 2-Hydroxynaphthalin-1-carbonsäure oder 6-Hydroxynaphthalin-1-carbonsäure,
Methylen-bis-hydroxybenzoesäuren, wie 5.5'-Methylen-bis-4-hydroxybenzoesäure,
Methylen-bis-hydroxynaphthoesäuren, wie Embonsäure,
Hydroxyzimtsäuren wie Ferulasäure,
Hydroxyhippursäuren wie Salicylursäure.

4. Verfahren zur Herstellung von Verbindungen der Formel I gemäß einem oder mehreren der Ansprüche 1-3, **dadurch gekennzeichnet, daß** man
ein in Wasser lösliches Salz der Formel I, beispielsweise ein Dihydrochlorid, Dihydrojodid oder ein Sulfat, mit Salzen der Carbonsäuren der Formeln II, III, IV oder V umsetzt, oder
ein Cephem-Betain der Formel VI 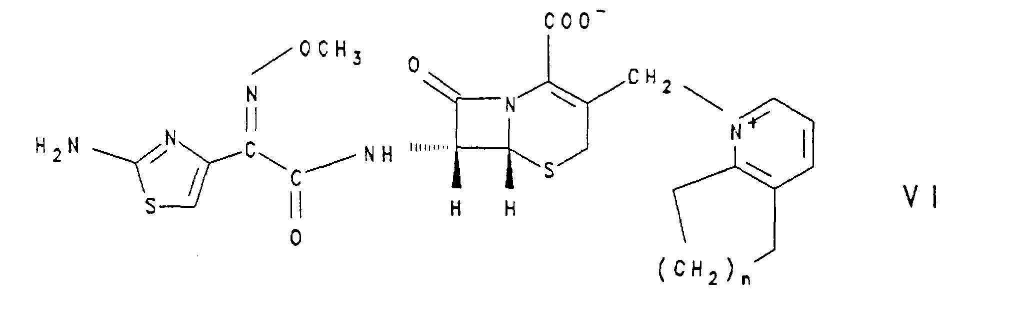
worin n die zu Formel I genannte Bedeutung hat, mit den Carbonsäuren der Formeln II, III, IV oder V umsetzt.

5. Verbindungen der Formel I gemäß einem oder mehreren der Ansprüche 1-3 zur Anwendung als Arzneimittel.

6. Verwendung von Verbindungen der Formel I zur Herstellung von Arzneimitteln zur Behandlung von bakteriellen Infektionen.

7. Arzneimittel, enthaltend eine wirksame Menge einer Verbindung der Formel I gemäß einem oder mehreren der Ansprüche 1-3.

8. Verfahren zur Herstellung eines Arzneimittels gemäß Anspruch 7, **dadurch gekennzeichnet, daß** eine Verbindung der Formel I gemäß einem oder mehreren der Ansprüche 1-3, ggf. mit geeigneten Trägerstoffen, Verdünnungsmitteln oder Puffersubstanzen in eine geeignete Darreichungsform gebracht wird.

## Claims

1. A compound of the formula I 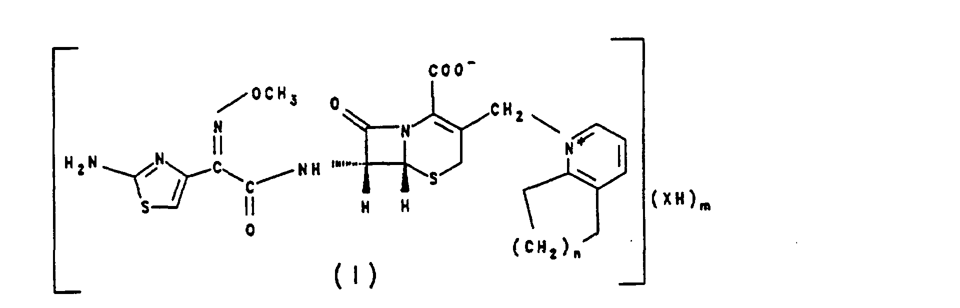 in which
n is equal to 1 or 2 and
m is 0.4 - 2.6, and where
X is the anion of a phenolic carboxylic acid of the formulae II a-d 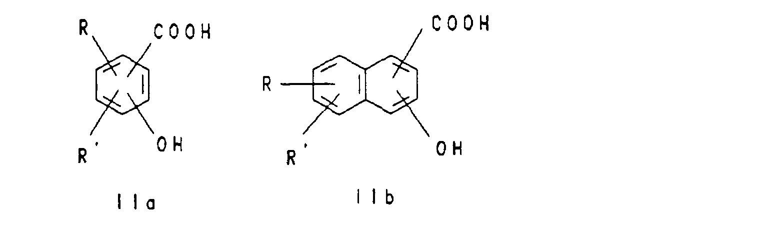 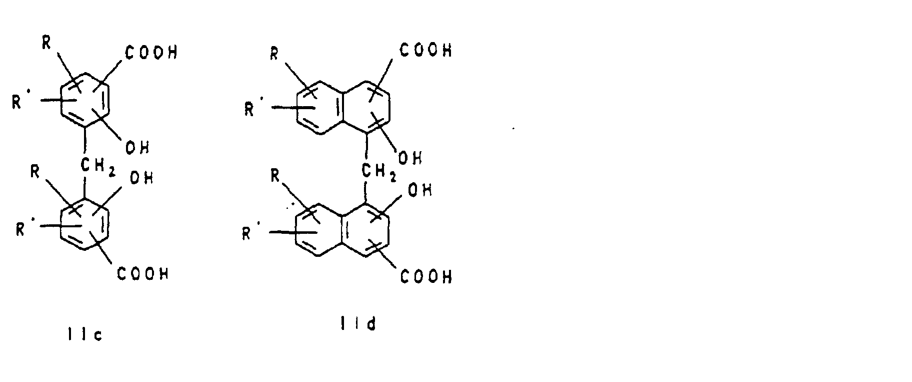
or the anion of a hydroxyphenylacetic acid of the formula III 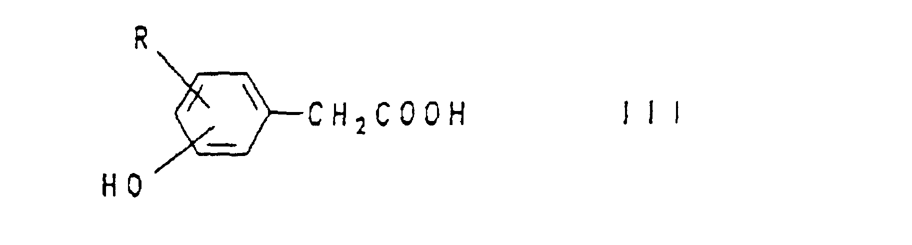 or the anion of a hydroxycinnamic acid of the formula IV 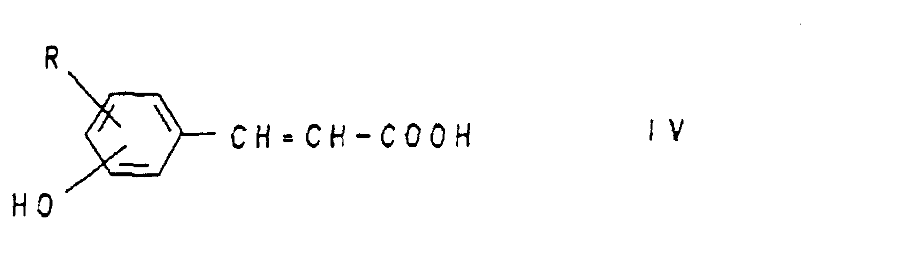 or the anion of a hydroxyhippuric acid of the formula V 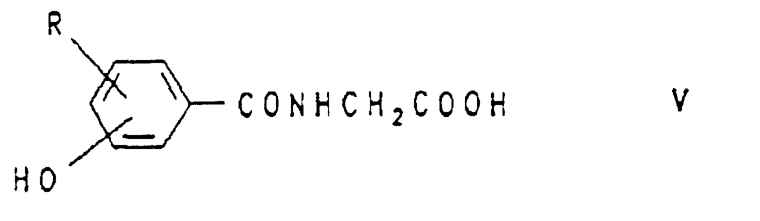 where R and R' independently of one another are hydrogen, carboxyl, hydroxyl, halogen, a straightchain or branched saturated or unsaturated aliphatic C₁-C₅-alkyl radical or C₁-C₅-alkyloxy radical and where X can also be X/2 in the case of a diprotic acid.

2. A compound of the formula I as claimed in claim 1, wherein X is the anion of a carboxylic acid of the formulae II-V, in which R and R' independently of one another are hydrogen, carboxyl, hydroxyl, methyl or methoxy and where m is 0.5-2.

3. A compound of the formula I as claimed in claim 1 or 2, wherein X is the anion of a compound selected from the following group of acids:
dihydroxybenzoic acids, such as 2,4-, 2,5- or 3,5-dihydroxybenzoic acid,
trihydroxybenzoic acids, such as gallic acid,
hydroxydicarboxylic acids, such as 4-hydroxyisophthalic acid,
hydroxynaphthalenecarboxylic acids, such as 2-hydroxynaphthalene-1-carboxylic acid or 6-hydroxynaphthalene-1-carboxylic acid,
methylenebishydroxybenzoic acids, such as 5,5'-methylenebis-4-hydroxybenzoic acid,
methylenebishydroxynaphthoic acids, such as embonic acid,
hydroxycinnamic acids such as ferulic acid,
hydroxyhippuric acids such as salicyluric acid.

4. A process for the preparation of compounds of the formula I as claimed in one or more of claims 1-3, which comprises
reacting a water-soluble salt of the formula I, for example a dihydrochloride, dihydroiodide or a sulfate, with salts of the carboxylic acids of the formula II, III, IV or V, or
reacting a cephem betaine of the formula VI 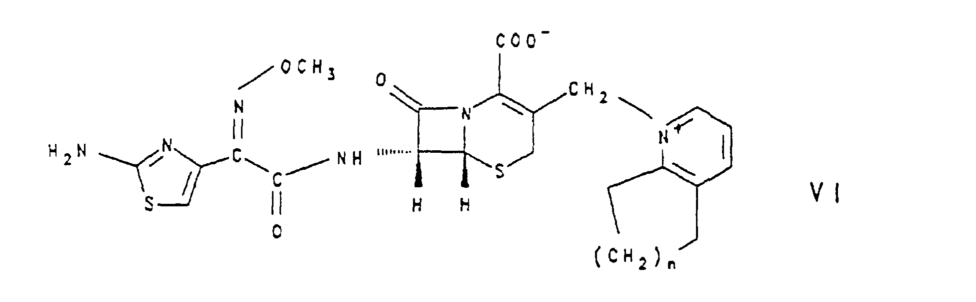
in which n has the meaning mentioned for formula I, with the carboxylic acids of the formula II, III, IV or V.

5. A compound of the formula I as claimed in one or more of claims 1-3 for use as a pharmaceutical.

6. The use of compounds of the formula I for the production of pharmaceuticals for the treatment of bacterial infections.

7. A pharmaceutical, containing an effective amount of a compound of the formula I as claimed in one or more of claims 1-3.

8. A process for the production of a pharmaceutical as claimed in claim 7, which comprises bringing a compound of the formula I as claimed in one or more of claims 1-3 into a suitable administration form, if appropriate using suitable excipients, diluents or buffer substances.

## Revendications

1. Composés de la formule I 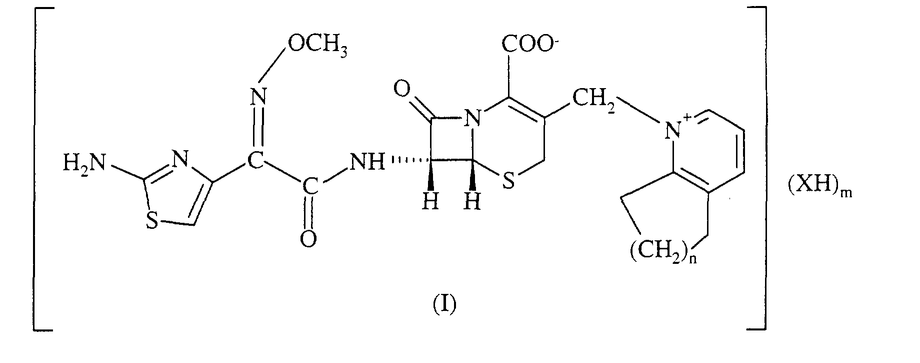 où
n est égal à 1 ou 2 et
m est 0,4-2,6 et
X représente l'anion d'un acide carboxylique phénolique des formules IIa-d 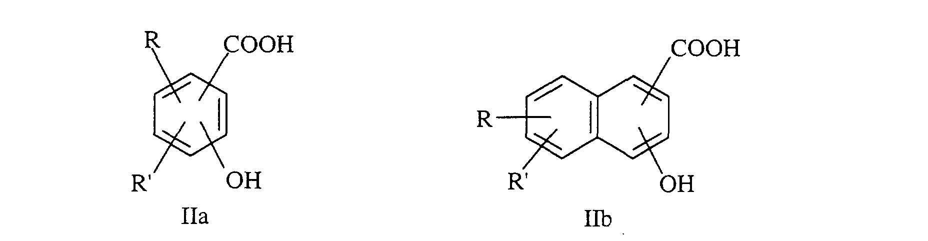 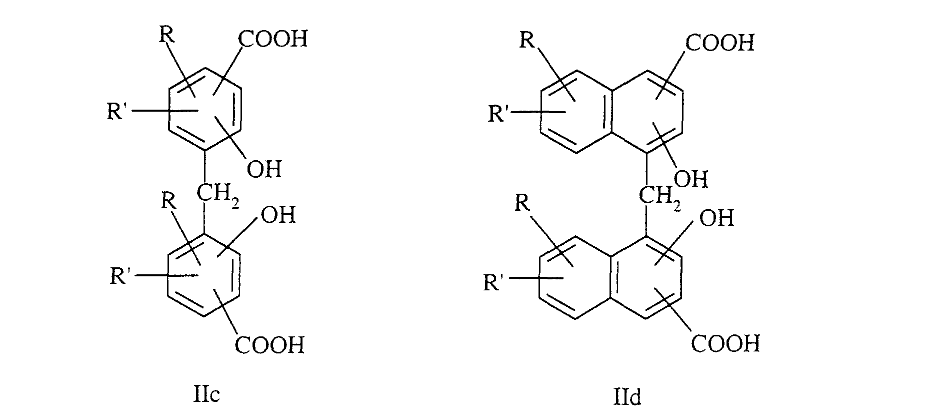
ou l'anion d'un acide hydroxyphénylacétique de la formule III, 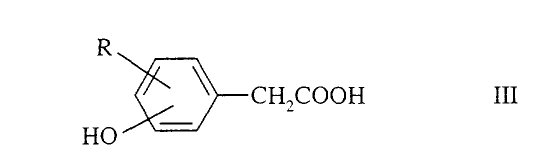 ou l'anion d'un acide hydroxycinnamique de la formule IV, 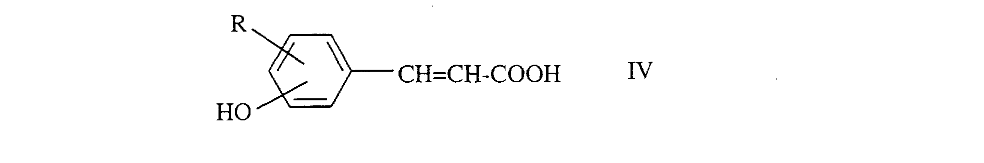 ou l'anion d'un acide hydroxyhippurique de la formule V, 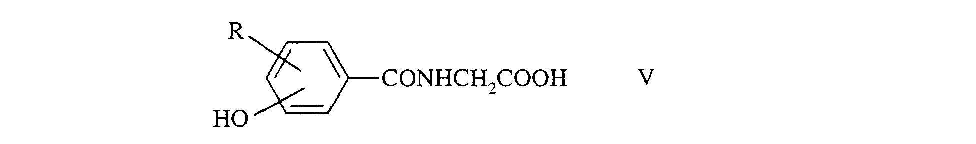 R et R' représentant indépendamment un atome d'hydrogène, un groupe carboxy, hydroxy, un atome d'halogène, un reste alkyle en C₁-C₅ ou un reste alkyloxy en C₁-C₅ aliphatique, linéaire ou ramifié, saturé ou insaturé et X pouvant également représenter X/2 dans le cas d'un acide bivalent.

2. Composés de la formule I selon la revendication 1, **caractérisés en ce que** X représente l'anion d'un acide carboxylique des formules II-V, dans lesquelles R et R' représentent indépendamment un atome d'hydrogène, un groupe carboxy, hydroxy, méthyle ou méthoxy et m étant compris entre 0,5-2.

3. Composés de la formule I selon les revendications 1 ou 2, **caractérisés en ce que** X est l'anion d'un composé choisi parmi les acides suivants :
les acides dihydroxybenzoïques, comme l'acide 2,4-, 2,5- ou 3,5-dihydroxybenzoïque,
les acides trihydroxybenzoïques, comme l'acide gallique,
les acides hydroxydicarboxyliques, comme l'acide 4-hydroxyisophtalique,
les acides hydroxynaphtalènecarboxyliques, comme l'acide 2-hydroxynaphtalène-1-carboxylique ou l'acide 6-hydroxynaphtalène-1-carboxylique,
les acides méthylène-bis-hydroxybenzoïques, comme l'acide 5,5'-méthylène-bis-4-hydroxybenzoïque,
les acides méthylène-bis-hydroxynaphtoïques, comme l'acide embonique,
les acides hydroxycinnamiques, comme l'acide férulique,
les acides hydroxyhippuriques, comme l'acide salicylurique.

4. Procédé pour la préparation de composés de la formule I selon une ou plusieurs quelconques des revendications 1-3, **caractérisé en ce que** l'on fait réagir un sel soluble dans l'eau de la formule I, par exemple un dihydrochlorure, un dihydroiodure ou un sulfate, avec des sels des acides carboxyliques des formules II, III, IV ou V, ou
on fait réagir une cephem-bétaïne de la formule VI 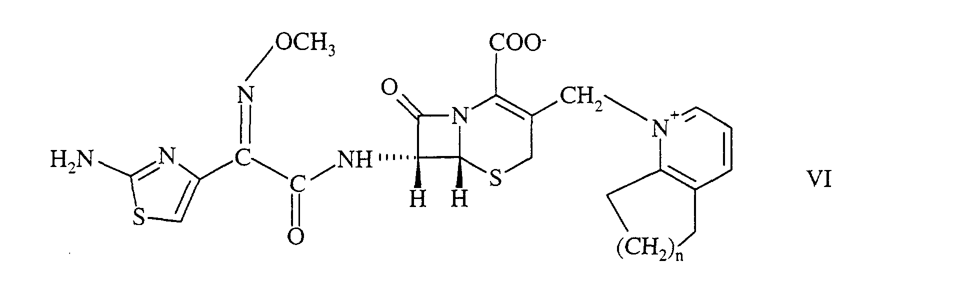 où n a la signification citée pour la formule I, avec les acides carboxyliques des formules II, III, IV ou V.

5. Composés de la formule I selon une ou plusieurs quelconques des revendications 1-3 pour une application comme médicament.

6. Utilisation de composés de la formule I pour la préparation de médicaments destinés au traitement d'infections bactériennes.

7. Médicament contenant une quantité efficace d'un composé de la formule I selon une ou plusieurs quelconques des revendications 1-3.

8. Procédé pour la préparation d'un médicament selon la revendication 7, **caractérisé en ce que** l'on met un composé de la formule I selon une ou plusieurs quelconques des revendications 1-3 dans une forme de présentation appropriée éventuellement avec des supports, des agents diluants ou des substances tampon appropriés.
